# EUROPEAN PATENT APPLICATION

(11) **EP 3 929 278 A1**
(43) Date of publication of application: **29.12.2021**
(21) Application number: 21169511.9
(22) Date of filing: 20.04.2021
(51) Int. Cl.: C12M 3/06, C12M 1/00, C12N 5/00, C12N 5/076, C12M 1/34, C12M 1/36, C12M 1/26

(54) **SAMPLE SEPARATING DEVICE AND METHOD**

(30) Priority: 24.06.2020 EP 20182102
(71) Applicant: University of Limerick, Limerick V94 T9PX (IE)
(72) Inventor: FAIR, Sean, Limerick, V94 T9PX (IE); NEWPORT, David, Limerick, V94 T9PX (IE); WHITE, Eoin, Limerick, V94 T9PX (IE); O'SULLIVAN, Kevin, Limerick, V94 T9PX (IE); O'SULLIVAN, Leonard, Limerick, V94 T9PX (IE)
(74) Representative: Appleyard Lees IP LLP

(57) **Abstract**

According to the present invention there is provided a sample separating device for separating motile organisms from other organisms, the device comprising: one or more primary fluid paths extending between a fluid inlet and a fluid terminus such that a fluid flow may be established between the fluid inlet and the fluid terminus; a sample introduction zone in the primary fluid path for introducing a sample to the primary fluid path; a sample collection zone for collecting a collected sample therein, the sample collection zone being located at a location upstream of the sample introduction zone in the primary fluid path; a sample collection zone outlet for facilitating removal of the collected sample from the sample collection zone; the sample separating device being arranged to allow communication with a sample moving system for moving the collected sample through the sample collection zone, thereby to move at least some of the collected sample from the sample collection zone to the sample collection zone outlet.

## Description

The present disclosure relates to a sample separating device and method, and in particular to a sample separating device for separating motile organisms from other organisms and a method of separating motile organisms from other organisms.

### BACKGROUND

In certain applications, it is desirable to separate motile organisms, such as sperm, from an original sample comprising a mixture of motile sperm, dead sperm, deformed sperm and other non-motile organisms. It may be desirable to separate the motile sperm from the other organisms in the sample as part of a preparation purpose for preparing sperm for an assisted fertilisation procedure, or simply for analysis.

An example of an assisted fertilisation procedure is artificial insemination which is also known as intrauterine insemination. Artificial insemination is a common procedure used in the field of livestock reproduction, and is also used as an infertility treatment for humans. Artificial insemination involves the introduction of sperm into a female's vagina, cervix or uterus by means other than sexual intercourse.

Another example of an assisted fertilisation procedure is in vitro fertilisation (IVF). IVF refers to a fertilisation procedure where an oocyte is combined with sperm outside of the body. In one example of this, a large number of sperm may be placed together with the oocyte and incubated so that fertilisation may take place. In another example of this, a single sperm may be injected directly into the oocyte in a process known as intra cytoplasmic sperm injection (ICSI). ICSI may be used, in particular, when the sperm donor has a low sperm count or low average sperm motility

In all of the above assisted fertilisation procedures, it is desirable to separate the motile sperm from the other organisms and detritus within the sample. This is so as to increase the chances of the fertilisation procedure being successful. This is particularly the case for ICSI, where an embryologist is required to select one sperm for injection into the oocyte. Providing a high-quality motile sperm sample with minimal sperm having abnormalities will make the selection process easier for the embryologist, and will reduce the failure rate of the ICSI procedure.

In the above described applications, a sample separating device is used to separate the motile organisms from other organisms in the original sample. An important design consideration for such a sample separating device are the features which relate to removal of a collected sample of separated motile organisms from the device. The method employed to remove the collected sample from the device is also important.

Existing designs of sample separating devices, and the methods typically employed, result in suboptimal removal of the collected sample of motile organisms.

In one example, the previously separated collected sample of motile organisms is subsequently contaminated by the other organisms during removal of the collected sample from the device. In another example, and in order to avoid contamination of the collected sample, the device must be extensively flushed or cleaned prior to the removal of the collected sample from the device. In a further example, involving a flushing or cleaning process, the device is provided with an arrangement of valves which are operated and controlled to prevent other organisms from contaminating the collected sample during the flushing, cleaning or removal processes.

The examples described above result in reduced sample quality and/or an increase in complexity of a sample separating device or the sample separating method.

It is desirable to improve on, or provide an alternative to, existing approaches for separating motile organisms from other organisms. Furthermore, it is desirable to improve on, or provide an alternative to, existing approaches for removal of a collected sample from a sample separating device.

It is an object of the present invention to improve on, or at least provide an alternative to, existing approaches for separating motile organisms form other organisms.

It is an object of the present invention to provide an improved device and/or method thereof and/or address one or more of the problems discussed above, or discussed elsewhere, or to at least provide an alternative device and/or method.

### SUMMARY OF THE INVENTION

According to the present invention there is provided a device, apparatus and method as set forth in the appended claims. Other features of the invention will be apparent from the dependent claims, and the description which follows.

According to a first aspect of the present invention there is provided a sample separating device for separating motile organisms from other organisms, the device comprising: one or more primary fluid paths extending between a fluid inlet and a fluid terminus such that a fluid flow may be established between the fluid inlet and the fluid terminus; a sample introduction zone in the primary fluid path for introducing a sample to the primary fluid path; a sample collection zone for collecting a collected sample therein, the sample collection zone being located at a location upstream of the sample introduction zone in the primary fluid path; a sample collection zone outlet for facilitating removal of the collected sample from the sample collection zone; the sample separating device being arranged to allow communication with a sample moving system for moving the collected sample through the sample collection zone, thereby to move at least some of the collected sample from the sample collection zone to the sample collection zone outlet.

In this way, a sample separating device is provided for the collection and removal of a high-quality sample of motile organisms. Sample separation by rheotaxis is facilitated by providing the sample collection zone upstream of the sample introduction zone in the primary fluid path. Providing a designated sample collection zone outlet simplifies removal of the collected sample, and minimises risk of contamination of the high-quality sample. By arranging the device to allow communication with a sample moving system, the complexity of the device can be reduced, whilst a dedicated sample moving system facilitates efficient removal of the sample by moving the collected sample from the sample collection zone to the sample collection zone outlet. Removal of the collected sample from the sample collection zone outlet, rather than directly from the sample collection zone, is advantageous as it mitigates or eliminates any risk of contamination of the collected sample, thus improving sample quality. In this way, the need to flush or otherwise clean the primary fluid path, where contaminants may be present, is also avoided. Overall, the system is mechanically simpler, yet more effective, than some prior art systems.

In one example, the sample separating device does not comprise valves or other mechanical means. In this way, flow from the sample introduction zone to the sample collection zone, and from the sample collection zone to the sample collection zone outlet, is performed without valves and is thus much simpler. This is facilitated by the advantageous location of the sample collection zone and arrangement of the sample separating device for rheotaxis. In prior art devices, valves or mechanical means are used to separate flow paths, or to disconnect or isolate flow paths, during a sample separating process. This is not the case here. The present device is thus much simpler in both construction and in operation.

In one example, the sample separating device may comprise one or more sample moving system connections. The sample moving system connections may be for facilitating connection with a sample moving system. In this way, a sample moving system may be connectable to the device for moving the collected sample through the sample collection zone, thereby to move at least some of the collected sample from the sample collection zone to the sample collection zone outlet.

In one example the device comprises a sample collection path defined by the sample collection zone extending through the sample collection zone to the sample collection zone outlet; and the sample separating device is arranged to allow communication with the sample moving system for moving the collected sample through the sample collection zone along the sample collection path.

That is, the sample collection zone may define a sample collection path extending along or through a part of, or all of, the sample collection zone. The sample collection path may extend to the sample collection zone outlet. In this way, removal of the sample from the sample collection zone outlet is facilitated. The sample separating device is arranged to allow communication with the sample moving system, which may be being arranged to receive or be connected with the sample moving system for moving the collected sample through the sample collection zone along the sample collection path. Providing a sample collection path in addition to the primary fluid path is advantageous as it minimises the risk of contamination of the collected sample, both during collection and during removal. In this way, a high-quality sample may be collected. The need to clean the device, by flushing the unsorted sample therefrom, prior to sample removal is also removed, by provision of both a primary fluid path, for use in sample separating, and an additional and distinct sample collection path, for use in collection and removal of the collected sample. That is, the primary fluid path and sample collection path may be described as being distinct, separate and/or at least partially non-interacting (e.g. they are distinct conduits). Nevertheless, the paths are providable in fluid communication with one another.

Moreover, in contrast with the prior art, sample removal is possible without any need to remove the unsorted sample prior to removal of a sorted sample. In this way, operation of the device is advantageously simplified

In one example at least a portion of the one or more primary fluid paths extends through a microchannel; the sample collection zone is in communication with the microchannel for receiving sample from the microchannel; and the sample collection path bypasses the at least a portion of the one or more primary fluid paths that extends through the microchannel.

The at least a portion of the one or more primary fluid paths extending through a microchannel is advantageous for a rheotaxis device. Motile organisms are able to swim up the microchannel against a fluid flow along the primary fluid path, and to the sample collection zone which is suitable for receiving, which may be arranged to receive, the sample from the microchannel. In this way, a collected sample of high-quality motile organisms can be collected in the sample collection zone. The sample collection path bypassing the at least a portion of the primary fluid path that extends through the microchannel is highly advantageous. By this construction, any need to clean or flush the device prior to sample collection is reduced and likely eliminated. This is because sample removal can take place in the absence of interaction with the primary fluid path, or at least the portion of the primary fluid path which extends through, or along, the microchannel. Efficient and effective sample removal is thereby facilitated, with a simple device arrangement.

In one example, the device may comprise a single microchannel. In another example. the device may comprise a plurality of microchannels. In one example, the device may comprise 10, 20, 30, 40, 50, 60, 70, 80, 90, 100, 200, 500, 1000, 2000, 5000 or more microchannels. In this way, a large quantity of high-quality collected sample may be collected in the sample collection zone. The microchannels may be radially arranged, for example all extending radially toward/away from a central zone. In this way, the size of the device may be reduced. The microchannels may be linearly arranged, for example all extending linearly toward/away from a central zone, the microchannels being parallel to one another. In this way, operation of the device may be simplified.

In one example the sample collection zone is arranged such that a hydraulic resistance of the sample collection path is less than an effective resistance of the microchannel (or, more generally, primary fluid path). In one example, the size, or volume, of the sample collection zone is greater than the combined size, or volume, of the microchannels (or, more generally, primary fluid path), such that a hydraulic resistance of the sample collection path is less than an effective resistance of the microchannel (or, more generally, primary fluid path).

This functionality means that the use of valves or other actuators may not be required in the flow path between the sample introduction and collection zones.

In this way, potential ingress of unseparated sample, and thereby contamination of the collocated sample, is minimised. In one example, the sample collection zone is 50 - 150mm in length, 1 - 5mm in width, and 0.2 - 1mm in depth. In one particularly advantageous example, the sample collection zone is 102mm in length, 2.65mm in width and 0.45mm in depth. In one example, the one or more microchannels may be 1 - 100mm in length. In a preferred example, the one or more microchannels may be 10mm in length. In one example, the width of the one or more microchannels may be in the range of 10um to 1000um, preferably between 50um and 600um, and most preferably 300um. In one example, the depth of each microchannel is between 10um to 300um, preferably 20um to 200um, and most preferably 100um. Such dimensions are examples that facilitate an arrangement wherein the hydraulic resistance of the sample collection path is less than an effective resistance of the combined microchannels. In this way, hydraulic resistance actuation is facilitated during sample collection. Other examples are possible, and may relate to the fluids that are used, and the environmental conditions (temperature, viscosity, etc.).

In one example the sample collection zone is arcuate.

In this way, a compact device construction is facilitated. A central collection zone can thereby be provided receiving a collected sample from numerous microchannels such that a large quantity of high-quality sample can be collected in a relatively small region.

In one example the sample separating device further comprises a sample extraction zone separated from the sample collection zone, the sample collection zone outlet being arranged to provide communication with the sample extraction zone.

In this way, sample may be moved to the sample collection zone outlet and into the sample extraction zone for extraction from the device. By providing the sample extraction zone separated from the sample collection zone, the risk of contaminating the collected sample with cells from the unsorted sample during removal of the collected sample from the device is reduced and likely eliminated. This ensures a high-quality collected sample. The sample extraction zone being separated from the sample collection zone may mean that the zones are distinct elements. Additionally the zones may be spatially separated, either laterally or vertically. Additionally the sample extraction zone may be isolated from the sample collection zone by virtue of being out of fluid communication with the sample collection zone in the absence of operation of the sample moving system.

In one example the sample separating device further comprises a fluid flow inhibiting element located between the sample collection zone and the sample extraction zone for inhibiting fluid flow therebetween in the absence of operation of the sample moving system. In one example the fluid flow inhibiting element is a wall separating the sample collection zone and the sample extraction zone. The wall may also be referred to as a barrier.

In this way, fluid flow between the sample collection zone and the sample extraction zone can be inhibited. Advantageously, operation of the sample moving system can allow the collected sample to move to the sample extraction zone. In this way, the high-quality collected sample can be isolated from the sample collection zone, and the rest of the device, as the extraction zone is separated from the collection zone. Extraction of the collected sample from the sample extraction zone then ensures that the collected sample is not contaminated. Sample removal is also simplified, as the sample can be readily removed from the sample extraction zone.

According to a second aspect of the present invention there is provided a sample separating apparatus for separating motile organisms from other organisms, the apparatus comprising: a sample separating device according to the first aspect of the present invention; and a sample moving system in communication with the sample separating device, the sample moving system being operable to move the collected sample through the sample collection zone, thereby to move at least some of the collected sample from the sample collection zone to the sample collection zone outlet.

The second aspect of the invention may comprise any or all of the features of the first aspect, as necessary or as desired.

Providing the sample moving system in addition to the sample separating device in a sample separating apparatus is highly advantageous. Moving the collected sample to the sample collection zone outlet facilitates efficient and effective removal of the sample from the device. Sample removal from the device may be performed by the sample moving system.

In one example the sample moving system is operable to flush a flushing medium through the sample collection zone, thereby to move at least some of the collected sample from the sample collection zone to the sample collection zone outlet. The sample moving system may comprise a centrifugal method, a pump or another method.

Flushing a flushing medium through the sample collection zone to move the collected sample to the sample collection zone outlet removes any need to remove the collected sample directly from the sample collection zone. In this way, risk of contamination of the sample by unsorted cells is minimised. The medium may be a sterile medium, or chosen as a medium which is easily separated from the collected sample, or does not impact the quality or usability of the collected sample. In one example the flushing medium is a liquid and/or gaseous medium.

In one example the sample moving system is operable to move the collected sample through the sample collection zone by inducing a pressure difference in the sample collection zone, thereby to move at least some of the collected sample from the sample collection zone to the sample collection zone outlet. In one example, the sample moving system is operable to pull the collected sample through the sample collection zone. In one example, the sample moving system is operable to push the collected sample through the sample collection zone. In one example, the sample moving system comprises a vacuum pump or pipette.

In this way the collected sample can be moved to the collection zone outlet for removal. This facilitates effective and efficient removal of the sample, whilst minimising contamination. Moreover, the apparatus construction is simple, in that only the sample collection zone needs be flushed or acted upon in order to remove the sample.

According to a third aspect of the present invention there is provided a method of separating motile organisms from other organisms, the method comprising: establishing a fluid flow along a primary fluid path between a fluid inlet and a fluid terminus; introducing a sample to the primary fluid path at a sample introduction zone; collecting a collected sample at a sample collection zone in the primary fluid path upstream of the sample introduction zone along the primary fluid path; arranging the sample separating device to allow communication with a sample moving system for moving the sample through the sample collection zone; and operating the sample moving system to move at least some of the collected sample from the sample collection zone to a sample collection zone outlet.

In this way, a method is provided for the collection and removal of a high-quality sample of motile organisms. Sample separation by rheotaxis is facilitated by collecting the collected sample upstream along the primary fluid path. Moving the sample to the sample collection zone outlet minimises risk of contamination of the high-quality sample by unsorted cells, for example by removing the sample directly from the sample collection zone. Moreover, moving the sample to sample collection zone outlet eliminates a need for flushing or otherwise cleaning the primary fluid path. The method is therefore simpler than prior art methods.

In one example the step of operating the sample moving system to move at least some of the collected sample from the sample collection zone to a sample collection zone outlet comprises: operating the sample moving system above a first threshold level to move at least some of the collected sample from the sample collection zone to the sample collection zone outlet and to a sample extraction zone, the sample extraction zone being separated from the sample collection zone.

In this way, the collected sample can be moved to a dedicated sample extraction zone, for extraction of the collected sample therefrom. This is highly advantageous in that the risk of contaminating the sample is reduced.

In one example the step of operating the sample moving system to move at least some of the collected sample from the sample collection zone to a sample collection zone outlet comprises: operating the sample moving system above a first threshold level to overcome a fluid flow inhibiting element.

In this way, the collected sample is moveable to the sample extraction zone where it will be fluidically isolated from the rest of the device in the absence of operation of the sample moving system.

In one example the method further comprises: operating the sample moving system below the first threshold level to fluidically isolate the at least some of the collected sample in the sample extraction zone from the sample collection zone.

In this way, the sample collection zone and sample extraction zone can be again fluidically isolated. The collected sample remains in the sample extraction zone, and can be extracted therefrom without the risk of contaminating the sample. This is because non-motile organisms in the rest of the device are prevented from being drawn into the sample extraction zone during removal as the sample extraction zone is fluidically isolated from the rest of the device. Efficient and effective removal of a high-quality sample of motile organisms is thereby facilitated.

### BRIEF DESCRIPTION OF THE DRAWINGS

For a better understanding of the invention, and to show how embodiments of the same may be carried into effect, reference will now be made, by way of example only, to the accompanying diagrammatic drawings in which:
Fig. 1 shows a sample separating device;
Fig. 2 shows a sample separating apparatus comprising the sample separating device of Fig. 1 and a sample moving system;
Fig. 3 shows a region of the sample separating device of Fig. 1;
Fig. 4.1 shows movement of a collected sample from a collection zone to an extraction zone;
Fig. 4.2 shows movement of a collected sample from a collection zone to an extraction zone;and
Fig. 5 shows general methodology principles.

### DETAILED DESCRIPTION

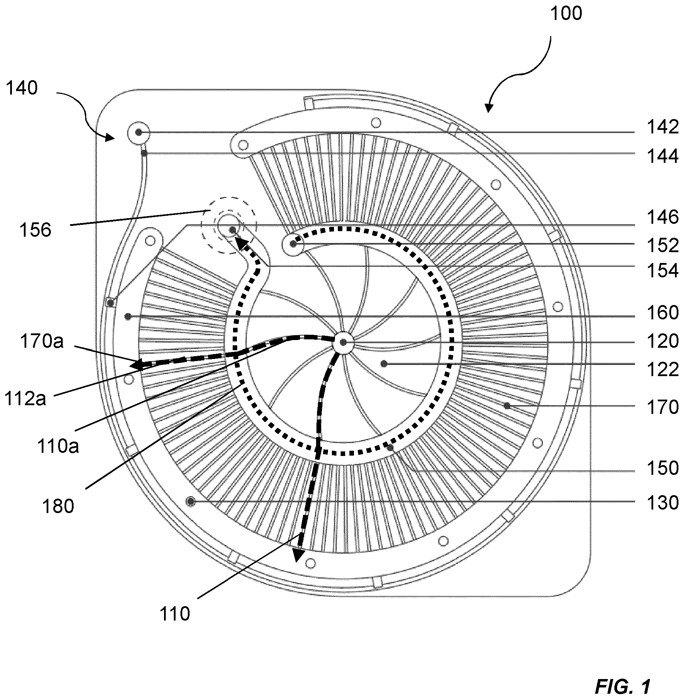

Referring to Figure 1, there is shown a sample separating device 100. The device 100 is in the form of a microfluidic chip. The device 100 is for separating motile organisms from other organisms.

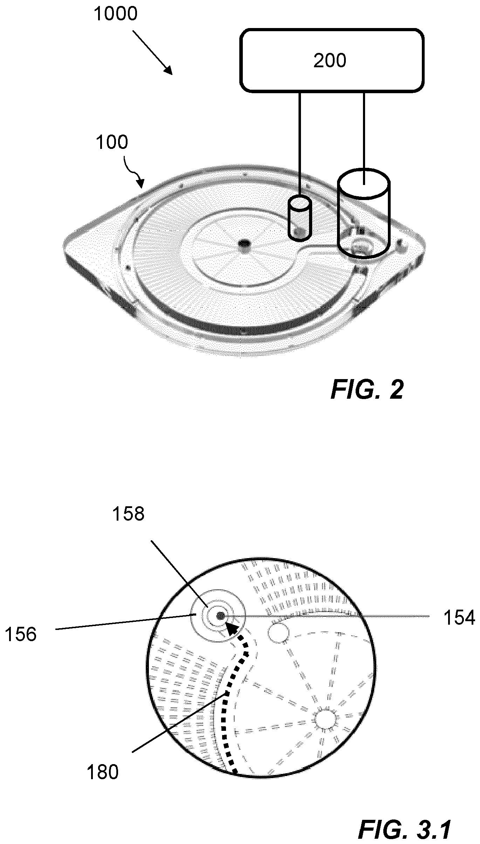

The device 100 comprises one or more primary fluid paths 110 extending between a fluid inlet 120 and a fluid terminus 130 such that a fluid flow may be established between the fluid inlet 120 and the fluid terminus 130. The device 100 comprises a sample introduction zone 140 in the primary fluid path 110 for introducing a sample to the primary fluid path 110. The device 100 comprises a sample collection zone 150 for collecting a collected sample therein, the sample collection zone 150 being located at a location upstream of the sample introduction zone 140 in the primary fluid path 110. The device 100 comprises a sample collection zone outlet 154 for facilitating removal of the collected sample from the sample collection zone 150. The device 100 is arranged to allow communication with a sample moving system 200 (shown in and described with reference to Figure 2) for moving the collected sample through the sample collection zone 150, thereby to move at least some of the collected sample from the sample collection zone 150 to the sample collection zone outlet 154.

Figure 1 shows a front surface of the device 100. At fluid inlet 120 a fluid may be introduced by a fluid delivery unit (not shown). The fluid terminus 130 is comprised in a plurality of fluid termini 130 provided in an outlet reservoir 160. As shown in the Figure, the fluid termini 130 are in fluid communication with the fluid inlet 120. It will be appreciated that whilst the fluid terminus 130 in this exemplary embodiment facilitate outlet from the device 100 in the outlet reservoir 160, they need not do so. Instead, the term fluid terminus 130 can be used simply to denote an end point of the primary fluid path 110. That is, the fluid terminus 130 may be any point in the outlet reservoir 160. A plurality of microfluidic channels, or "microchannels" 170 extend radially outwardly from the central sample collection zone 150, the zone 150 being in the form of an arc. Each microchannel 170 is defined by a pair of channel walls. By providing a plurality of microchannels 170 a greater quantity of collected sample may be obtained by the separating device 100 and separating method.

The sample introduction zone 140 is provided in the form of sample inlet 142 and loading channel 144. The loading channel 144 is an arc surrounding the outlet reservoir 160. The sample introduction zone 140 denotes a region of the device 100 where a sample may be introduced. This is typically performed by pipetting the sample intro the sample inlet 142 and allowing it to disperse through the loading channel 144. The sample introduction zone 140 thus allows for uniform distribution of the sample about the microchannels 170. Therefore, there is an increased probability of collecting a high-quality sample by the device 100 and method, as the motile organisms are able to be distributed throughout the loading channel 144 where they can enter the outlet reservoir 160 via a plurality of diffusion channels 146 to access nearby microchannels 170.

The sample collection zone 150 denotes a region of the device 100 where motile organisms may be collected. The sample collection zone 150 is located upstream of the sample introduction zone 140. That is, in use, fluid flows from the sample collection zone 150 to the sample introduction zone 140 downstream, such that motile organisms exhibiting rheotaxis swim from the sample collection zone 150 upstream. In the example of Figure 1, the sample collection zone 150 is provided at the terminal ends of the microchannels 170.

In an example operation of the device of Figure 1, a fluid source such as a syringe may be connected to the fluid inlet 120. The syringe may be activated by a fluid delivery unit such as a syringe pump. Initially, during a priming phase, fluid may be delivered into the fluid inlet 120 at a high, device priming flow velocity. The fluid will flow from the fluid inlet 120 into and through the microchannels 170. The fluid exiting the microchannels 170 will then flow to the fluid termini 130.

Notably, fluid flowing from the fluid inlet 120 travels into and through a plurality of radially extending flow distribution channels 122. Each flow distribution channel 122 is arcuate, and together the flow distribution channels 122 have a spiral-like form extending outwardly from the fluid inlet 120. Fluid flowing out of the flow distribution channels 122 has a high mean flow velocity, compared with the mean flow velocity through the microchannels 170. This is a result of the fewer flow distribution channels 122 compared with the number of microchannels 170. In this way, motile organisms that have collected in the sample collection zone 150 are prevented from entering the flow distribution channels 122.

Notably, the fluid flow is uniformly distributed between microchannels 170, such that a substantially identical flow rate is present in each microchannel 170. Furthermore, during priming, fluidic jets are induced in the outlet reservoir 160 at the ends of the microchannels 170 by the high device priming flow velocity. The motile organisms swim toward the jets but are prevented from entering the microchannels 170 by the high flow velocity.

Once the device 100 is primed, the flow velocity of the fluid flow is reduced to the sample introduction flow velocity. A sample is then introduced into the sample introduction zone 140. The sample introduction flow velocity prevents the sample entering the microchannels 170 or sample collection zone 150 by mechanisms such as capillary action or due to the pipetting force.

Again, the higher mean flow velocity prevents the motile organisms from entering the microchannels 170.

The flow velocity is then reduced again to the operational flow velocity, and motile organisms in the sample are able to swim against the fluid flow, swim up the microchannels 170 to the sample collection zone 150.

Significantly, when the flow rate is reduced to the operational flow velocity, fluidic jets resulting from the higher mean flow velocity cease to force the motile organisms away from the microchannels 170, and instead, in combination with dimensions of the channels 170 or channel openings, induce 3-D counter rotating vortices in the outlet reservoir 160 proximal to the opening of the microchannels 170. These vortices aid transportation of the motile organisms from the sample towards the openings of the microchannels 170. Thus, these vortices encourage motile organisms to the openings of the microchannels 170. These vortices might alternatively or additionally be described as the introduction and maintenance of turbulence proximal to the opening of the microchannels 170.

The flow velocity in the sample collection zone 150 is at a level that motile organisms that enter the sample collection zone 150 remain therein and are not forced back down microchannels 170. Again, as mentioned above, the higher mean flow velocity of fluid flow from flow distribution channels 122 prevents motile organisms from entering the flow distribution channels 122.

The flow velocity is then increased to a sample collection flow velocity. A sample collection step is then performed. Whilst increasing the flow velocity to a sample collection flow velocity is advantageous in preventing other organisms entering the sample collection zone, the skilled person will nevertheless appreciate that this is not essential to the invention. Instead, and for example, the flow velocity may be reduced to zero.

Sample collection can be achieved as described herein. The skilled person will appreciate that benefits can be obtained by performing the sample collection step in absence of any of the teaching above, including the discussion of flow control. The sample collection step may be referred to as a flush collection step.

The sample collection step, or flush collection step, makes use of the sample collection zone inlet 152 and sample collection zone outlet 154. The sample collection zone inlet 152 and sample collection zone outlet 154 are in fluid communication with the sample collection zone 150. Fluid may be flushed from the sample collection zone inlet 152 to the sample collection zone outlet 154, thus removing from the sample collection zone 150 the motile organisms that were able to propagate up the microchannels 170 to the sample collection zone 150.

The sample collection zone 150 defines a sample collection path 180. The sample collection path 180 is a path along which a collected sample may be moved along in order to remove or extract the collected sample, or facilitate removal or extraction of the collected sample, from the device 100. In this exemplary embodiment, the sample collection zone 150 is arcuate, and thus defines an arcuate sample collection path 180. The sample collection path 180 extends through the sample collection zone 150 to the sample collection zone outlet 154. As illustrated in Figure 1, the sample collection path 180 extends from the sample collection zone inlet 152 through the sample collection zone 150 to the sample collection zone outlet 154.

Referring now to Figure 2, but still with reference to Figure 1, a sample separating apparatus 1000 is schematically shown. The apparatus 1000 is for separating motile organisms from other organisms. The apparatus 1000 comprises a sample separating device 100 as described herein. The apparatus 1000 further comprises a sample moving system 200. The sample moving system 200 is for moving the collected sample through the sample collection zone 150. In this way, the sample moving system 200 can move at least some of the collected sample from the sample collection zone 150 to the sample collection zone outlet 154. The sample moving system 200 could take the form of a syringe, pipette, pump, or other device that is able to move the sample.

The sample separating device 100 is arranged to allow communication with the sample moving system 200. The device 100 comprises sample moving system connection points in the region of, or proximal to, the sample collection zone inlet 152 and sample collection zone outlet 154. The connection points facilitate connection of the sample moving system 200 to the device 100. By connection of the system 200 to the device 100, the system 200 is in communication, that is, fluid communication, with the device 100.

The sample moving system 200, in communication with the sample separating device 100, is operable to move the collected sample through the sample collection zone 150, thereby to move at least some of the collected sample from the sample collection zone to the sample collection zone outlet 154. The sample moving system 200 may be operated in a number of different ways in order to move the collected sample through the sample collection zone 150.

In one mode of operation, the sample moving system 200 is operable to pull the collected sample through the sample collection zone 150 to the sample collection zone outlet 154. This may be achieved by inducing a pressure difference in the sample collection zone 150, such as by virtue of a vacuum pump, syringe, pipette, or other suitable means.

In another mode of operation, the sample moving system 200 is operable to push the collected sample through the sample collection zone 150 to the sample collection zone outlet 154. This may be achieved by operating the sample moving system 200 to introduce a flushing medium, such as a liquid and/or gaseous medium, into the sample collection zone 150, in a region of or at the sample collection zone inlet 152, and operating the sample moving system to flush the flushing medium through the sample collection zone 150. In this way, the collected sample is pushed to the sample collection zone outlet 154.

In yet another mode of operation, the sample moving system 200 is operable to both push and pull the collected sample through the sample collection zone 150 using a combination of the operating modes described above.

By the above described modes of operation, and possible alternative modes, the sample moving system 200 can move at least some of the collected sample from the sample collection zone 150 to the sample collection zone outlet 154.

Referring back to Figure 1, and to the description above, the one or more primary fluid paths 110 extending between the fluid inlet 120 and fluid terminus 130 allow a fluid flow to be established between the fluid inlet 120 and the fluid terminus 130 along the primary fluid path 110. A first primary fluid path 110a is illustrated in Figure 1. As shown, a portion 112a of the first primary fluid path 110a extends through a microchannel 170a, from the sample collection zone 150 to the outlet reservoir 160. The sample collection zone 150 is in communication with the microchannel 170a for receiving sample from the microchannel 170a. The sample collection path 180 bypasses the portion 112a of the first primary fluid path 110a that extends through the microchannel 170a. That is, the primary fluid paths 110 and the sample collection path 180 are separate, or may be described as being at least partially non-interacting. In this way, there is no need to flush or clean the microchannels 170 in order to remove the unsorted sample from the device, where removal of the sample is facilitated by movement of the collected sample along the sample collection path 180 as will be described further herein.

The sample collection zone 150 is arranged such a hydraulic resistance of the sample collection path 180 is less than an effective resistance of the microchannels 170, such that ingress of the collected sample into the microchannels 170 is substantially prevented when the collected sample is moved through the sample collection zone 150 by the operation of the sample moving system 200, as described above. For the avoidance of doubt, hydraulic resistance refers to a single (that is, one) channel or path. Effective resistance refers to the overall resistance of the microchannels 170. The effective resistance looks at the combined resistance of the microchannels 170 and can be assessed with a single value, which can be compared with the hydraulic resistance value of the sample collection path 180. In an example, the resistance of a single microchannel 170 will be large, resulting in limited flow through said microchannel 170. However, due to the large number of microchannels 170, the effective resistance of the sum of the microchannels 170 is considerably less. It is useful to compare the hydraulic resistance of the sample collection path 180 with the effective resistance of all microchannels 170 in order to make a useful comparison which can allow optimum sizing and shaping (i.e. arrangement) of the sample collection zone 150. Each microchannel length may range from 1-100mm, and the width of each microchannel can range from 10um to 1000um, with a preferred range of 50um to 600um. In this way, the ratio of resistances are optimised to prevent ingress of collected sample into the microchannels 170. This is because the fluid flow will be dominated by viscous effects and inertial contributions will be minimised. Consequently, hydraulic resistances may be employed to model the flow. A preferred example comprises a microchannel length of 10mm, a microchannel width of 300um, and a microchannel depth of 100um.

Referring to Figure 3.1, the sample separating device 100 further comprises a sample extraction zone 156. In this exemplary embodiment, the sample extraction zone 156 is in the form of an extraction well. The sample extraction zone 156 is an annular well extending around the sample collection zone outlet 156 and is concentric therewith. The sample collection zone outlet 154 is arranged to provide communication with the sample extraction zone 156, so that the sample collection zone outlet 154 feeds into the sample extraction zone 156. The sample extraction zone 156 is separated from the sample collection zone 150. In this way, the sample collection zone outlet 154 allows collected sample to be fed into the sample extraction zone, but held separately from the sample collection zone 150. Collected sample can therefore be removed from the sample extraction zone 156 independently of (that is, without interaction with) the sample collection zone.

The sample collection zone 150 and sample extraction zone 156 are separated from one another by a fluid flow inhibiting element 158. The fluid flow inhibiting element 158 inhibits fluid flow from the sample collection zone 150 to the sample extraction 156 in the absence of a driving force, or a flushing force, acting on the collected sample in the sample collection zone 150 to move the collected sample along the sample collection path 180. As described above, this force is provided by operation of the sample moving system 200.

The fluid flow inhibiting element 158 is in the form of an annular barrier wall 158 extending upwardly about the sample collection zone outlet 154. The height of the barrier wall 158 is such that its uppermost extent is higher than a lowermost extent of the well 156.

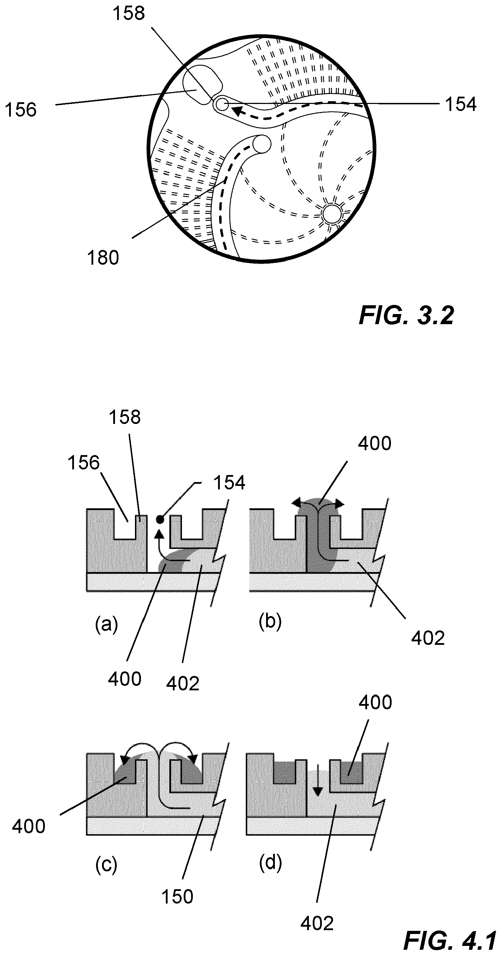

Referring to Figure 3.2, in another exemplary embodiment, the sample separating device 100 also comprises a sample extraction zone 156 is the form of an extraction well. However, in this exemplary embodiment, the sample extraction zone 156 is a spatially separated well provided adjacent to the sample collection zone outlet 156. The sample collection zone outlet 154 is arranged to provide communication with the sample extraction zone 156, so that the sample collection zone outlet 154 feeds into the sample extraction zone 156. As above, the sample extraction zone 156 is separated from the sample collection zone 150. In this way, the sample collection zone outlet 154 allows collected sample to be fed into the sample extraction zone, but held separately from the sample collection zone 150. Collected sample can therefore be removed from the sample extraction zone 156 independently of (that is, without interaction with) the sample collection zone.

The sample collection zone 150 and sample extraction zone 156 are separated from one another by a fluid flow inhibiting element 158. The fluid flow inhibiting element 158 inhibits fluid flow from the sample collection zone 150 to the sample extraction 156 in the absence of a driving force, or a flushing force, acting on the collected sample in the sample collection zone 150 to move the collected sample along the sample collection path 180. As described above, this force is provided by operation of the sample moving system 200.

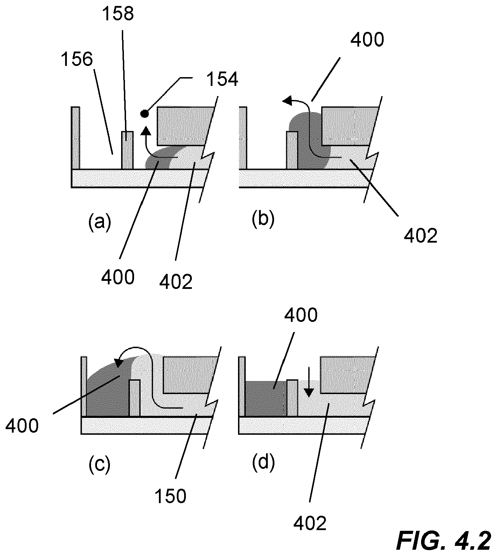

The fluid flow inhibiting element 158 is in the form of a substantially linear barrier wall 158 extending upwardly and defining a part of the sample collection zone outlet 154. The height of the barrier wall 158 is such that its uppermost extent is higher than the height of the sample collection zone 150, as shown in Figure 4.2.

Referring to Figures 4.1 and 4.2, in combination with Figures 1 and 2, movement of a collected sample 400 from the sample collection zone 150 to the sample extraction zone 156 is shown. Such movement is facilitated by operation of the sample moving system 200.

As shown in Figure 4.1 (a) and 4.2(a), a flushing medium 402, in this example a liquid flushing medium, is introduced into the sample collection zone 150 at the sample collection zone inlet 152. The sample moving system 200 is operated at a first level to flush the flushing medium 402 through the sample collection zone 150. This forces the collected sample 400 along the sample collection path 180 toward the sample collection zone outlet 154.

As shown in Figure 4.1(b) and 4.2(b), the sample moving system 200 continues to operate at the first level to move the collected sample 400 to the sample collection zone outlet 154. Continued operation forces the collected sample 400 upwardly from the outlet 154. Operation of the sample moving system 200 is sufficient to overcome the fluid flow inhibiting element 158, and thus the collected sample 400 is pushed up and over the barrier wall 158.

As shown in Figure 4.1(c) and 4.2(c), the collected sample 400 falls over the barrier wall 158 and into the sample extraction zone 156. The collected sample 400 is collected in the sample extraction zone 156.

As shown in Figure 4.1(d) and 4.2(d), once some or all of the collected sample 400 is collected in the sample extraction zone 156, operation of the sample moving system 200 is reduced to a second level. At this level of operation of the sample moving system 200, the force is insufficient to overcome the fluid flow inhibiting element 158. The fluid flow inhibiting element 158 once again inhibits fluid flow from the sample collection zone 150 to the sample extraction zone 156, and the sample extraction zone 156 and sample collection zone 150 are fluidically isolated.

The collected sample 400 which has been moved to the sample extraction zone 156 can then be extracted from the sample extraction zone 156. This may be achieved by operating the sample moving system 200 to pull the collected sample 400 from the sample extraction zone 156, without pulling any of the sample 400 or flushing medium 402 remaining in the sample collection zone 150. Alternatively, extraction of the collected sample from the sample extraction zone 156 may be achieved by pipetting from the sample extraction zone 156. It will be understood that extracting the collected sample 400 from the sample extraction zone 156, rather than from the sample collection zone 150, is advantageous as it ensures that only the high-quality collected sample is extracted from the device 100. In this way, the device 100 and apparatus 1000 mitigates any risk of other organisms (which may be non-motile, or insufficiently motile, sperm) present in the microchannels 170 being inadvertently extracted from the device 100 during extraction.

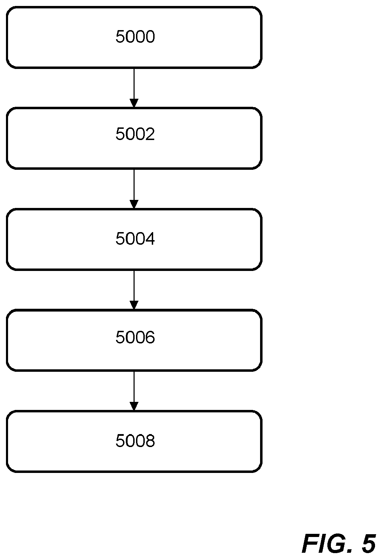

Referring to Figure 5, there is shown a method of separating motile organisms from other organisms. Step 5000 comprises establishing a fluid flow along a primary fluid path between a fluid inlet and a fluid terminus. Step 5002 comprises introducing a sample to the primary fluid path at a sample introduction zone. Step 5004 comprises collecting a collected sample at a sample collection zone in the primary fluid path upstream of the sample introduction zone along the primary fluid path. Step 5006 comprises arranging the sample separating device to allow communication with a sample moving system for moving the sample through the sample collection zone. Step 5008 comprises operating the sample moving system to move at least some of the collected sample from the sample collection zone to a sample collection zone outlet.

In summary, an advantageous sample separating device, apparatus and method is described. Collection and removal of a high-quality sample of motile organisms is made possible. Sample separation by rheotaxis is facilitated by providing the sample collection zone upstream of the sample introduction zone in the primary fluid path. The arrangement of the device, apparatus and method eliminates any need to flush or otherwise clean the microchannels of the device prior to sample removal. Flushing the collected sample along the sample collection path mitigates risk of contaminating the sample. Many advantages and benefits of the presently described device, apparatus and method will be apparent to the skilled person from the present disclosure and the drawings.

Although a few preferred embodiments of the present invention have been shown and described, it will be appreciated by those skilled in the art that various changes and modifications might be made without departing from the scope of the invention, as defined in the appended claims.

The preceding description with reference to the accompanying drawings is provided to assist in a comprehensive understanding of various embodiments of the disclosure as defined by the claims and their equivalents. It includes various specific details to assist in that understanding but these are to be regarded as merely exemplary. Accordingly, those of ordinary skill in the art will recognize that various changes and modifications of the various embodiments described herein can be made without departing from the scope and spirit of the disclosure. In addition, descriptions of well-known functions and constructions may be omitted for clarity and conciseness.

The terms and words used in the preceding description and claims are not limited to the bibliographical meanings, but, are merely used by the inventor to enable a clear and consistent understanding of the disclosure. Accordingly, it should be apparent to those skilled in the art that the following description of various embodiments of the disclosure is provided for illustration purpose only and not for the purpose of limiting the disclosure as defined by the appended claims and their equivalents.

It is to be understood that the singular forms "a," "an," and "the" include plural referents unless the context clearly dictates otherwise. The terms "front", "rear", "side", "upper", "lower", "over", "under", "inner", "outer" and like terms are used to refer to the apparatus and its components in the orientation in which it is illustrated, which is the orientation in which it is intended to be used but should not be taken as otherwise limiting. Like reference numerals are used to denote like features throughout the figures, which are not to scale.

At least some of the example embodiments described herein may be constructed, partially or wholly, using dedicated special-purpose hardware. Terms such as 'component', 'module' or 'unit' used herein may include, but are not limited to, a hardware device, such as circuitry in the form of discrete or integrated components, a Field Programmable Gate Array (FPGA) or Application Specific Integrated Circuit (ASIC), which performs certain tasks or provides the associated functionality. In some embodiments, the described elements may be configured to reside on a tangible, persistent, addressable storage medium and may be configured to execute on one or more processors. These functional elements may in some embodiments include, by way of example, components, such as software components, object-oriented software components, class components and task components, processes, functions, attributes, procedures, subroutines, segments of program code, drivers, firmware, microcode, circuitry, data, databases, data structures, tables, arrays, and variables. Although the example embodiments have been described with reference to the components, modules and units discussed herein, such functional elements may be combined into fewer elements or separated into additional elements. Various combinations of optional features have been described herein, and it will be appreciated that described features may be combined in any suitable combination. In particular, the features of any one example embodiment may be combined with features of any other embodiment, as appropriate, except where such combinations are mutually exclusive. Throughout this specification, the term "comprising" or "comprises" means including the component(s) specified but not to the exclusion of the presence of others.

Attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings) may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extends to any novel one, or any novel combination, of the features disclosed in this specification (including any accompanying claims, abstract and drawings), or to any novel one, or any novel combination, of the steps of any method or process so disclosed.

## Claims

1. A sample separating device for separating motile organisms from other organisms, the device comprising:
one or more primary fluid paths extending between a fluid inlet and a fluid terminus such that a fluid flow may be established between the fluid inlet and the fluid terminus;
a sample introduction zone in the primary fluid path for introducing a sample to the primary fluid path;
a sample collection zone for collecting a collected sample therein, the sample collection zone being located at a location upstream of the sample introduction zone in the primary fluid path;
a sample collection zone outlet for facilitating removal of the collected sample from the sample collection zone;
the sample separating device being arranged to allow communication with a sample moving system for moving the collected sample through the sample collection zone, thereby to move at least some of the collected sample from the sample collection zone to the sample collection zone outlet.

2. A sample separating device as claimed in claim 1 wherein the device comprises:
a sample collection path defined by the sample collection zone extending through the sample collection zone to the sample collection zone outlet; and
the sample separating device is arranged to allow communication with the sample moving system for moving the collected sample through the sample collection zone along the sample collection path.

3. A sample separating device as claimed in claim 2 wherein:
at least a portion of the one or more primary fluid paths extends through a microchannel;
the sample collection zone is in communication with the microchannel for receiving sample from the microchannel; and
the sample collection path bypasses the at least a portion of the one or more primary fluid paths that extends through the microchannel.

4. A sample separating device as claimed in claim 3 wherein the sample collection zone is arranged such that a hydraulic resistance of the sample collection path is less than an effective resistance of the microchannel.

5. A sample separating device as claimed in any previous claim further comprising a sample extraction zone separated from the sample collection zone, the sample collection zone outlet being arranged to provide communication with the sample extraction zone.

6. A sample separating device as claimed in claim 5 further comprising a fluid flow inhibiting element located between the sample collection zone and the sample extraction zone for inhibiting fluid flow therebetween in the absence of operation of the sample moving system.

7. A sample separating device as claimed in claim 6 wherein the fluid flow inhibiting element is a wall separating the sample collection zone and the sample extraction zone.

8. A sample separating apparatus for separating motile organisms from other organisms, the apparatus comprising:
a sample separating device as claimed in any previous claim; and
a sample moving system in communication with the sample separating device, the sample moving system being operable to move the collected sample through the sample collection zone, thereby to move at least some of the collected sample from the sample collection zone to the sample collection zone outlet.

9. A sample separating apparatus as claimed in claim 8 wherein the sample moving system is operable to flush a flushing medium through the sample collection zone, thereby to move at least some of the collected sample from the sample collection zone to the sample collection zone outlet.

10. A sample separating apparatus as claimed in claim 9 wherein the flushing medium is a liquid and/or gaseous medium.

11. A sample separating apparatus as claimed in any of claims 8 to 10 wherein the sample moving system is operable to move the collected sample through the sample collection zone by inducing a pressure difference in the sample collection zone, thereby to move at least some of the collected sample from the sample collection zone to the sample collection zone outlet.

12. A method of separating motile organisms from other organisms, the method comprising:
establishing a fluid flow along a primary fluid path between a fluid inlet and a fluid terminus;
introducing a sample to the primary fluid path at a sample introduction zone;
collecting a collected sample at a sample collection zone in the primary fluid path upstream of the sample introduction zone along the primary fluid path;
arranging the sample separating device to allow communication with a sample moving system for moving the sample through the sample collection zone; and
operating the sample moving system to move at least some of the collected sample from the sample collection zone to a sample collection zone outlet.

13. A method as claimed in claim 12 wherein the step of operating the sample moving system to move at least some of the collected sample from the sample collection zone to a sample collection zone outlet comprises:
operating the sample moving system above a first threshold level to move at least some of the collected sample from the sample collection zone to the sample collection zone outlet and to a sample extraction zone, the sample extraction zone being separated from the sample collection zone.

14. A method as claimed in claim 13 wherein the step of operating the sample moving system to move at least some of the collected sample from the sample collection zone to a sample collection zone outlet comprises:
operating the sample moving system above a first threshold level to overcome a fluid flow inhibiting element.

15. A method as claimed in either of claims 13 or 14 further comprising:
operating the sample moving system below the first threshold level to fluidically isolate the at least some of the collected sample in the sample extraction zone from the sample collection zone.
